# EUROPEAN PATENT SPECIFICATION

(11) **EP 1 030 653 B1**
(45) Date of publication and mention of the grant of the patent: **09.11.2005**
(21) Application number: 99937118.0
(22) Date of filing: 17.08.1999
(51) Int. Cl.: A61K 9/127, A61K 31/136, A61P 33/02

(54) **DINITROANILINE LIPOSOMAL FORMULATIONS AND PROCESSES FOR THEIR PREPARATION**
DINITROANILIN ENTHALTENDE LIPOSOMEN UND VERFAHREN ZUR HERSTELLUNG DERSELBEN
DOSAGE LIPOSOMAUX A BASE DE DINITROANILINE ET LEURS PROCEDES DE PREPARATION

(30) Priority: 21.08.1998 PT 10219798
(43) Date of publication of application: 30.08.2000
(73) Proprietor: INSTITUTO NACIONAL DE ENGENHARIA E TECNOLOGIA INDUSTRIAL/INSTITUTO DE BIOTECNOLOGIA, QUIMICA FINA E TECNOLOGIAS ALIMENTARES, 1699 Lisboa Codex (PT)
(72) Inventor: MEIRINHOS DA CRUZ, Maria, Eugénia, P-1500 Lisboa (PT); CARVALHEIRO, Manuela, Colla, P-1150 Lisboa (PT); JORGE, Joao Carlos Santana, P-1800 Lisboa (PT)
(74) Representative: Alves Moreira, Pedro
(86) International application number: PCT/PT1999/000015
(87) International publication number: WO 2000/010532

(56) References cited:
- WO-A-86/01102
- WO-A-95/31970
- GENNARO AR, ET AL (EDS): "Remington's Pharmaceutical Sciences, 17th ed." 1985 , MACK PUBLISHING CO., EASTON, USA , ISBN: 0-912734-03-5 XP002127988 page 1644 -page 1661 & LONGER MA AND ROBINSON JR: "Sustained-release drug delivery systems" passage "Liposomes" page 1659 -page 1660
- CHAN MM, ET AL.: "Herbicides to curb human parasitic infections: In vitro and in vivo effects of trifluralin on the trypanosomatid protozoans" PROCEEDINGS OF THE NATIONAL ACADEMY OF SCIENCES OF THE USA, vol. 90, no. 12, 1993, pages 5657-5661, XP002127876 ISSN: 0027-8424 cited in the application

## Description

### Field of Invention

This invention relates to liposomal compositions containing one dinitroaniline, incorporated or encapsulated for use in the preparation of a pharmaceutical formulation for treatment in humans and animals and to processes for their preparation.

The referred liposomal formulations can contain as dinitroaniline, for example, preferably, trifluralin (TFL).

Besides dinitroanilines, the liposomal formulations of the invention contain also phospholipids, individually or in mixtures, hydrogenated or not, with or without cholesterol (Chol) and electrically charged molecules, lipidic or not, as, for example, phosphatidylinositol (PI), phosphatidylglycerol (PG), dioleoylphosphatidylglycerol (DOPG), stearylamine (SA).

### Invention background

The diseases caused by intracellular parasites of the mononuclear phagocytic system (MPS) cells are among the most important diseases all over the world due to the number of cases annually reported. One of these diseases is leishmaniasis, caused by a haemoflagellate protozoan named, in general, *Leishmania sp.* This disease has an incidence of at least 12 million infections in humans and animals. The dog has a crucial role as reservoir of the protozoan, being one among the responsible by the maintenance of zoonose. The disease is propagated from reservoirs to humans by vectors (sandflies). Leishmaniasis represents an immense public health problem in the Middle East, Africa, India, China, Central and South America, and other tropical and subtropical areas throughout the world like the Mediterranean region including Portugal (33, 34).

Leishmaniasis, depending on the subspecies, can assume several forms of the disease: visceral, mucocutaneous and cutaneous. The visceral form of the disease is usually fatal if not treated. All forms may be linger and recurrent despite the treatment with pentavalent antimonial compounds, the recommended first choice drugs (20, 26, 33, 34).
*Leishmania sp* are able to live in the mononuclear phagocytic system cells, in an intracellular vesicle inside the host cell (2, 5, 14, 16, 31). The fusion of host cell Iysosomes with the vacuole containing the parasite, does not prevent the *leishmania* multiplication. This fusion can even supply the necessary nutrients for its multiplication. In this way, the parasite seems to be safe inside the cell, being this one of the reasons why its elimination is so difficult. This fusion mechanism lysosome-vacuole can be used for alternative therapies namely through the internalisation mechanism of liposomes by MPS cells (2).

Several different classes of drugs have been used to treat leishmaniasis, namely pentavalent antimonial compounds, trivalent antimonial compounds, antibiotics (polyenics, aminoglucosides), immunomodulators (interferon α) and chelating agents (desferrioxiamine), among several others (26, 30, 32, 35).

The present recommended treatment for canine leishmaniasis is a course of pentavalent antimonial drugs, either sodium stibogluconate or meglumine antimoniate. These drugs have a limited effectiveness and they do not achieve a complete cure of the disease. These therapies are accompanied by a combination of problems, particularly: variable efficacy, long course of treatments and severe side effects such as cardiac and renal toxicities. Increased resistance to treatment with pentavalent antimonial drugs has also been reported and attributed to inadequate treatments (9, 20, 24, 26, 32, 33, 35).

Other drugs, used in the treatment of leishmaniasis, have limited clinic application also due to severe side effects, such as amphotericin B that is nephrotoxic (14, 20) and methotrexate (MTX) with cardiac toxicity (10, 11).

In spite some progresses in the development of new drugs have been achieved, none has 100% success in the treatment of the disease. Besides the referred toxic effects, the small efficacy of treatments is the other main disadvantage of the used drugs against infections by *Leishmania sp* (14).

As examples of these drugs it can be referred methotrexate (85% reduction on the infection level) (10, 11) pentamidine (less efficacy than antimonial derivatives), dehydroemetine, achieving 67% of cure (1) and desferrioxiamine, with 44% efficiency (32). In the cases of treatment failure with pentavalent antimonials, subsequent treatments with other classes of drugs, such as pentamidine, amphotericin B, ketoconazole and paramomycin, do not significantly increase the results. Also formycin B, sinefungin and lepidine WR6026 showed high antileishmanial activity when compared to pentavalent antimonials, but toxicity problems persist (26, 27).

Allopurinol and related compounds (allopurinol nucleoside, thiopurinol, thiopurinol ribonucleoside) have been tested *in vitro* and *in vivo* (27). The protozoans are not able to synthesise purines, being dependent on host purines and nucleosides. The presence of inosine analogues (e.g. allopurinol ribonucleoside) inhibits the purine metabolising enzymes of the parasite, affecting RNA function and reducing protein synthesis (26, 27). Previous studies showed that allopurinol, allopurinol nucleoside and thiopurinol ribonucleoside have small activity in animal models of the disease, probably due to the small residence time and low serum levels obtained by these drugs (27).

Antibiotics, such as, streptomycin and trobamycin inhibit the growth of both promastigote and amastigote forms of the parasite (25).

Trifluralin is a herbicide known to be active against leishmaniasis. This drug binds to plant tubulins but not to animal tubulins. *Leishmania sp* tubulins are very similar to plant tubulins. In this way, trifluralin showed to be able to inhibit promastigote proliferation, to reduce promastigote to amastigote transformation, to interfere with amastigote replication and to reduce amastigote infectivity. *In vitro* studies confirmed efficiency against all forms of leishmaniasis, but *in vivo* studies only presented good results against the cutaneous forms of the disease. A drug delivery system need to be developed for the use of trifluralin against visceral forms since trifluralin solubility and lipophilicity do not allow the administration by any other route than the topical one. By this route no activity against the visceral forms was observed (12).

In view of the difficulties above described, an alternative approach to the search of new drugs is the drug encapsulation in macrophage directed carriers, as liposomes.

Liposomes are phospholipid synthetic bilayer vesicles able of incorporating a variety of substances independently of their molecular weight, electrical charge and solubility (18, 19, 23).

The rationale for the use of liposomal associated drugs instead of free drugs for the treatment of visceral leishmaniasis rely on the fact that amastigotes of the parasite are specifically located in liver spleen and bone marrow macrophages. As liposomes are preferentially taken up by these cells (2, 5, 30), they can deliver toxic agents straight to the intracellular location of established parasites (28). Though, the administration of liposome-encapsulated agents theoretically increases the therapeutic index of the agent in two ways: 1) increasing the uptake of the carrier and consequently of the drug by macrophages contained organisms, and 2) reducing toxicity of the free drug due to relatively low uptake of carrier by organs to which the drug is toxic (2, 6, 16, 20).

The great majority of drug delivery systems administered by intravenous route are taken up from circulation by the liver, meaning that they accumulate preferentially in this organ, not achieving, at significant quantities, other organs also belonging to MPS (spleen and bone marrow). The uptake by these other organs can be increased by the reduction on vesicle diameter (8). This can result in the suppression of the infection in the spleen and bone marrow, quite difficult to achieve with the free drugs.

Results in literature show that liposome encapsulated drugs are much safer and more effective to treat MPS infection as compared to free drugs (5, 17, 28).

Liposomal formulations of pentavalent antimonials can increase 200 to 700 times the therapeutic index compared with the free form, depending upon the lipid composition of liposomes (4, 7, 8, 13). Liposomal amphotericin B is 2 to 5 times more active than free form (6, 29). Liposomal primaquine presents activity at doses not actives for the free form (3). However these results are strongly dependent from the infection stage at the beginning of treatment and are difficult to correlate due to the heterogeneity of the experimental conditions (15).

Most of the above described results suffer from limitations of the kind of liposomes used, with low encapsulation efficiency and of small half lives, not reaching crucial targets for the treatment of this disease and, also, because of high costs.

WO 95/31970 teaches a method for the production of liposomal microencapsulated agricultural agents with one of these agents being trifluralin. However the described method refers to processes of production of a saturated organic solution of lipid and an active agent that, upon addition of water, would become a liposomal formulation. By definition, liposomes are lipid vesicles consisting on one or more concentric sealed bilayers, dispersed in an aqueous environment. The presence of organic solvents will turn impossible the formation of a bilayer structure. Several important aspects of this method make it not appropriate for any pharmaceutical purpose. The presence of the organic solvent in the final preparation is one of them. Also, the concentration and purity of the used lecithin would not be appropriate.

WO 86/01102 teaches a method for loading liposomes with an ionizable antineoplastic agent or with a variety of other ionizable drugs. These authors make reference of the use of sugars as protective agents for the dehydration-rehydration process in order to keep liposomes intact as the water in the system is removed. However there is still the problem on avoiding sublimation of dinitroanilines, particularly of trifluralin, as they make no reference on the use of any molecule, including sugars, as anti-sublimating agents during all manipulation processes and not only for lyophilization protection.

Liposomal amphotericin B was effective in curing a few cases of visceral leishmaniasis in humans (16, 21, 22), but the cost of such a treatment is too high for widely application to animals infected population.

### Invention Detailed Description

The present invention refers to liposomal formulations containing one dinitroaniline and to processes for their preparation.

The present invention concerns the achievement, under stable form, lyophilised or not, of liposomal formulations containing one dinitroaniline, for example trifluralin, incorporated or encapsulated.

In the formulations obtained according to the present invention, the liposomal diameter varies between 0,01 µm to 50 µm. According to the present invention, a mixture of two different size populations exist in the formulations, with diameters respectively bigger and lower than 100 nm.

Additionally the present invention formulations may contain any of the following lipids, hydrogenated or not, individually or in mixtures, in any molar ratio: distearoylphosphatidylcholine (DSPC), phosphatidylcholine (PC), cholesterol (Chol) or derivatives, sphingomielin (SM), dioleoylphosphatidylcholine (DOPC), dioleoylphosphatidylglycerol (DOPG), phosphatidylglycerol (PG), dimiristoylphosphatidylcholine (DMPC), dipalmitoylphosphatidylcholine (DPPC), gangliosides, ceramides, phosphatidylinositol (PI), phosphatydic acid (PA), dicetylphosphate (DcP), dimiristoylphosphatidylglycerol, (DMPG), stearylamine (SA), dipalmitoylphosphatidylglycerol (DPPG) and other synthetic lipids.

The preparation process of liposomal formulations of the present invention comprises the steps of:
- obtention of the liposomal formulations containing vesicles of dinitroaniline by hydration, with a solution containing an antisublimating agent of a lipid film containing the dinitroaniline
- obtention of the different populations with well-defined diameters by a sizing step
- mixing the obtained distinct populations;
- lyophilization/dehydration of the so obtained liposomal formulation; and
- rehydration of the dehydrated liposomal formulation

In a common way, solubilization in organic solvent of the lipidic components and the dinitroaniline, for example trifluralin, can be done, followed by drying under N₂ stream or under vacuum, for example, in a rotavapor with controlled temperature for the achievement of a mixed homogeneous film of lipid and dinitroaniline, for example, trifluralin. This film can be, subsequently, hydrated with a sugar solution forming multillamelar liposomes. The following step can be the liposomal formulation sizing, under pressure, by successive extrusions through polycarbonate membranes of pore sizes varying from 5,0 to 0,01 µm. The sizing will end preferably after extrusion through the membrane with the desired pore size for a part of the population. After the attainment of the necessary different populations with well-determined diameter, the following step is the mixture of these populations.

After the attainment of the necessary different populations of well-determined diameter, the following step is the mixture of these populations. After the mixture of the populations, it can be, or not, done a concentrative dialysis, using, for example, polyethyleneglycol as hygroscopic agent, followed by a step of dehydration. This dehydration occurs preferably in the presence of sugars that will act as protective of sublimation of the dinitroaniline, for example, trifluralin.

The formulations according the present invention so obtained, after hydration with water, are ready for use.
Up to now, there is no literature reference to liposomal preparations with dinitroanilines.

According the present invention, in order to prepare the multillamelar liposomes a step of drying a mixture of one dinitroaniline, namely trifluralin, and lipids, both solubilized in the same solvent or mixture of organic solvents, is performed. The amount of trifluralin varies according the final volume to prepare, ranging from 10 µg to 1 g or more. The amount of lipid also changes according the final volume to be prepared, ranging from 1 µmole to 1 mole or more. The adequate lipids, hydrogenated or not for the preparation of the formulations are present individually or in mixtures, in any molar ratio from the following lipids: distearoylphosphatidylcholine (DSPC), phosphatidylcholine (PC), cholesterol (Chol) or derivatives, sphingomielin (SM), dioleoylphosphatidylcholine (DOPC), dioleoylphosphatidylglycerol (DOPG), phosphatidylglycerol (PG), dimiristoylphosphatidylcholine (DMPC), dipalmitoylphosphatidylcholine (DPPC), gangliosides, ceramides, phosphatidylinositol (PI), phosphatydic acid (PA), dicetylphosphate (DcP), dimiristoylphosphatidylglycerol, (DMPG), stearylamine (SA), dipalmitoylphosphatidylglycerol (DPPG) and other synthetic lipids.
The so obtained mixture is submitted to a step of drying under a N₂ stream, until the total remove of the solvent or mixture of solvents. After drying, hydration of the mixture with a solution of a sugar as, for example, trehalose, is done, ranging its concentration from 0,01 M to 2 M, under mechanical stirring or manual external stirring. The so obtained liposomal formulation is, then, submitted to a step of sizing, for example, by successive passages under pressure through polycarbonate filters of decreasing pore diameter, normally referred as extrusion. Extrusion starts normally through 5 µm diameter pore membranes and continues with passages through diameter pore membranes of 2, 1, 0,8, 0,6, 0,4, 0,2, 0,1 and 0,05 µm, reaching some times 0,02 µm. According to a preferred way preparation of the invention and after passage through 0,4 µm membranes, the so obtained liposomal preparation is split in two parts. Only one of those parts goes through the rest of the extrusion procedure until, for example, 0,05 µm diameter pore membranes. At the end, the two parts that correspond to two distinct populations are mixed, achieving, by this way, one liposomal formulation containing liposomes that exhibit two different diameter distribution populations. The simultaneous presence of these different diameter populations present the advantage that, after *in vivo* parenteral administration, the population of bigger diameter is rapidly captured by mononuclear phagocytic system cells, while the small size population remains in circulation, possibly reaching organs other than liver and spleen, where the parasitic infection also exist, as for example, the bone marrow.

The so obtained formulation may be, or not, submitted to a step of concentration by dialysis against, for example, polyethyleneglycol that will act as a water removing agent. After this dialysis step, the formulation can be frozen up to -70°C during, at least one hour, after what it is submitted to lyophilization.

After this lyophilization, the formulation is ready to be used, being enough for that, the addiction of water to the so obtained powder. Hydration occurs instantaneously originating one homogeneous suspension in water of liposomes, containing the dinitroaniline as, for example, trifluralin.

A particularly preferred way of preparation of the present invention is the one in which to a lipid mixture of DOPC:DOPG in a molar ratio of 7 : 3, in a total of 10 µmole of lipid, solubilized in chloroform, is added 1 µmole of trifluralin, solubilized in chloroform. The obtained mixture is, then, dried under a stream of nitrogen until total evaporation of the chloroform. The so film is hydrated with 0,1 mL of 0,3 M trehalose, with manual stirring. After complete resuspension of the lipidic film, the formulation rests for 15 minutes, after what 0,1 mL more of the same trehalose solution is added. Another 15 minutes resting period is allowed after what hydration is completed by adding 0,8 mL of the same solution. The so obtained liposomal formulation is submitted to a sizing step by passage under pressure through polycarbonate membranes of successively decreasing pore diameters, from 5 µm to 0,4 µm. After this extrusion procedure, the formulation is divided in two equal parts. One of those parts continues the sizing step until a filter of 0,05 µm. The two populations so obtained are, then, mixed. The mixed liposomal formulation is submitted to freezing at -70°C for 60 minutes and, after that period, lyophilised. In this way, a liposomal formulation ready to be hydrated with 1,0 mL of distilled sterile water is obtained, able to be parenterically administered.

The formulations may also contain auxiliary substances, pharmaceutically acceptable, useful for preservation of their quality and or to turn them closely related to physiological conditions, such as pH adjusting agents, buffering agents, tonicity agents, antioxidants and other adjuvants as, for example, sodium acetate, sodium lactate, sodium chloride, potassium, chloride, calcium chloride, glucose, saccharose, mannitol, xylitol, alpha-tocopherol.

The pharmaceutical formulations obtained according the present invention can be administered to warm blood animals, such as man, already suffering from leishmaniasis, during the necessary time interval and in a necessary quantity to end or significantly inhibit infection progress. The adequate quantities for the achievement of that effect are named as "therapeutically efficient doses". The therapeutic efficient doses for this use will depend on the infection degree and on the general state of health of the treatment individual. There is no other formulation of the free drug, namely, trifluralin, used in parenteric administration.

The following examples, of liposomal formulations prepared according the present invention and of their respective physico-chemical and biological analysis, are presented as illustrations and not as limitations.

### Literature

1. Al-Kateeb, G.H., Molan, A.L. (1981) "Efficacy of some drugs on *Leishmania donovani* in the Golden Hamster, mesocricetus auratus" Chemother. 27, 117-125.
2. Alving, C.R. (1983) "Delivery of liposome-encapsulated drugs to macrophages" Pharmac. Ther. 22, 407-424.
3. Alving, C.R., Steck, E.A., Chapman Jr, W.L., Waits, V.B., Hendricks, L.D., Swartz Jr, G.M., Hanson, W.L. (1980) "Liposomes in leishmaniasis: therapeutic effects of antimonial drugs, 8-aminoquinolines and tetracycline" Life Sciences 26, 2231-2238.
4. Alving, C.R., Steck, E.A., Chapman Jr., W.L., Waits, V.B., Hendricks, L.D., Swartz Jr., G.M., Hanson, W.L. (1978) "Therapy of leishmaniasis: superior efficacies of liposome-encapsulated drugs" Proc. Natl. Acad. Sci. USA 75, 2959-2963.
5. Alving, C.R., Steck, E.A., Hanson, W.L., Loizeaux, P.S., Chapman Jr, W.L., Waits, V.B. (1978) "Improved therapy of experimental leishmaniasis by use of a liposome-encapsulated antimonial drug" Life Sciences 22, 1021-1026.
6. Berman, J.D., Hanson, W.L., Chapman, W.L., Alving, C.R., Lopez-Berestein, G. (1986) "Antileishmanial activity of liposome-encapsulated amphotericin B in hamsters and monkeys" Antimicrob. Agents Chemother. 30, 847-851.
7. Black, C.D.V., Watson, G.J. (1977) "The use of Pentostam liposomes in the chemotherapy of experimental leishmaniasis" Trans. R. Soc. Trop. Med. Hyg. 71, 550-552.
8. Carter, K.C., Dolan, T.F., Alexander, J., Baillie, A.J., McColgan, C. (1989) "Visceral leishmaniasis: drug carrier system characteristics and the ability to clear parasites from the liver, spleen and bone marrow in *Leishmania donovani* infected BALB/c mice" J. Pharm. Pharmacol. 41, 87-91.
9. Carter, K.C., O'Grady, J., Dolan, T.F., Baillie, A.J., Alexander, J., Keys, J. (1989) "A direct comparison of sodium stibogluconate treatment in two animal models of human visceral leishmaniasis, mouse and hamster" Int. J. Pharm. 53, 129-137.
10. Chakraborty, P., Bhaduri, A.N., Das, P.K. (1990) "Neoglycoproteins as carriers for receptor-mediated drug targeting in the treatment of experimental visceral leishmaniasis" J. Protozool. 37, 358-364.
11. Chakraborty, P., Bhaduri, A.N., Das, P.K. (1990) "Sugar receptor mediated drug delivery to macrophages in the therapy of experimental visceral leishmaniasis" Biochem. Biophys. Res. Comm. 166, 404-410.
12. Chan, M.M.-Y., Grogl, M., Chen, C.-C., Bienen, E.J., Fong, D.(1993) "Herbicides to curb human parasitic infections: *In vitro* and *in vivo* effects of trifluralin on the trypanosomatid protozoans" Proc. Natl. Acad. Sci. USA 90, 5657-5661.
13. Chapman Jr., W.L., Hanson, W.L., Alving, C.R., Hendricks, L.D. (1984) "Antileishmanial activity of liposome-encapsulated meglumine antimoniate in the dog" Am. J. Vet. Res. 45, 1028-1030.
14. Croft, S., (1989) "Recent advances in anti-protozoal chemotherapy" Actual. Chim. Ther. 20, 57-70.
15. Croft, S., Neal, R.A., Rao, L. (1989) "Liposomes and other drug delivery systems in the treatment of leishmaniasis" pp. 783-792; in "Leishmaniasis: the currents status and new strategies for control", ed. by Harte, D.T., New York: Plenum Press.
16. Croft, S.L., Davidson, R.N., Thornton, E.A. (1991) "Liposomal amphotericin B in the treatment of visceral leishmaniasis" J. Antimicrob. Chemother. 28, 111-118.
17. Croft, S.L., Hogg, J., Gutteridge, W.E., Hudson, A.T., Randall, A.W. (1992) "The activity of hydroxynaphthoquinones against *Leishmania donovani*" J. Antimicrob. Chemother. 30, 827-832.
18. Cruz, M.E., Corvo, M.L., Jorge, J.S., Lopes, F. (1989) "Liposomes as carrier systems for proteins: factors affecting protein encapsulation" pp. 417-426, in "Liposomes in the Therapy of Infectious Diseases and Cancer", ed by Lopez-Berestein G. and Fidler I.J., New York: Alan R. Liss, Inc.
19. Cruz, M.E.M., Gaspar, M.M., Lopes, F., Jorge, J.S., Perez-Soler, R. (1993) "Liposomal L-asparaginase: in vitro evaluation" Int. J. Pharm. 16, 67-77.
20. Davidson, R.N., Croft, S.L. (1993) "Recent advances in the treatment of visceral leishmaniasis" Trans. R. Soc. Trop. Med. Hyg. 87, 130-31, 141.
21. Dupla, M.L., Aguado, A.G., Uriol, P.L., Garcia, V.P., Ortega, E.V., Martinez, P.M., Garciapuig, J. (1993) "Efficacy of liposomal amphotericin-B in the treatment and secondary prophylaxis of visceral leishmaniasis in HIV infected patients - report of two cases" J. Antimicrob. Chemother. 32, 657-659.
22. Giacchino, R., Giambartolomei, G.,Tasso, L., Timitilli, A., Castagnola, E., Brisigotti, M., Micalizzi, C. (1993) "Treatment with liposomal amphotericin B of a child affected with drug-resistant visceral leishmaniasis" Trans. R. Soc. Trop. Med. Hyg. 87, 310.
23. Gregoriadis, G. (1991) "Overview of liposomes." J. Antimicrobial Chemotherapy 28S, 39-48.
24. Madindou, T.J., Hanson, W.L., Chapman Jr, W.L. (1985) "Chemotherapy of visceral leishmaniasis (*Leishmania donovani*) in the squirrel monkey (Samiri sciureurs)" Annals Trop. Med. Parasitol. 79, 13-19.
25. Navin, T.R., Pearson, R.D., (1987) "Inhibition of *Leishmania donovani* growth by streptomycin and tobramycin" Ann. Trop. Med. Parasitol. 81, 6, 731-733.
26. Neal, R.A. (1987) "Experimental chemotherapy" pp. 793-845, *in* The Leishmaniasis. Vol II, London: Academic Press, Inc.
27. Neal, R.A., Croft, S.L., Nelson, D.J. (1985) "Anti-leishmanial effect of allopurinol ribonucleoside and the related compounds, alluporinol, thiopurinol, thiopurinol ribonucleoside, and of formycin B, sinefungin and the lepidine WR6062" Trans. Royal Society Trop. Med. Hyg. 79, 122-128.
28. New, R.R.C., Chance, M.L., Thomas, S.C., Peters, W. (1978) "Antileishmanial activity of antimonials entrapped in liposomes" Nature 272, 55-56.
29. Ramos, H., Romero, E., Cohen, B.E. (1988) "The differential effect of liposomal amphotericin B on human erythrocytes and promastigotes of *Leishmania sp*." Acta Cient. Venezolana 39, 135-139.
30. Reed, S.G., Barral-Netto, M., Inverso, J.A. (1984) "Treatment of experimental visceral leishmaniasis with Iymphokine encapsulated in liposomes" J. Immunol. 132, 3116-3119.
31. Rees, P.H., Kager, P.A. (1987) "Visceral leishmaniasis and post-kala-azar dermal leishmaniasis" in: The Leishmaniasis in Biology and Medicine, ed. by: W. Peters and R. Killick-Kendrick, Vol. 2, 584-615, Academic Press, London.
32. Segovia, M., Navarro, A., Artero, J.M. (1989) "The effect of liposome-entrapped desferrioxamine on Leishmania donovani in vitro" Ann. Trop. Med. Parasitol. 83, 357-360.
33. WHO Expert Committee on the Control of Leishmaniases (1984) 'The Leishmaniases: Report of a WHO Expert Committee" vol. 701, in WHO Technical Report Series, ed. by World Health Organization, Geneva.
34. WHO Expert Committee on the Control of Leishmaniases (1990) "Control of leishmaniases: Report of a WHO Expert Committee" vol. 793, in WHO Technical Report Series, ed. by World Health Organization, Geneva.
35. Zumla, A., Croft, S.L. (1992) "Chemotherapy and immunity in opportunistic parasitic infections in AIDS" Parasitology 105, S93-S101.

### Examples

These examples illustrate liposomal formulation prepared according to the present invention and processes for their preparation in which the used dinitroaniline is trifluralin.

### Trifluralin (TFL) incorporation in liposomes

The preparation of the following described formulations, in a total volume of 5 mL for lipidic composition, started by the addition of TFL to lipid in chloroform, followed by evaporation of the solvent under nitrogen stream. Hydration of the resulting film was done by adding 500 µL of trehalose 0,3 M, stirring and resting for 15 minutes, addition of 500 µL more of 0,3 M trehalose, stirring again and resting again for a new 15 minute period and, finally, by the addition of 4000 µL of 0,3 M trehalose. Samples for dosage (initial TFL and initial lipid) were removed. The liposomal formulations so obtained were sized by successive filtration, under nitrogen pressure, through polycarbonate filters with pores of 5,0, 2,0, 1,0, 0,8, 0,6 and 0,4 µm, with two passages in the last filter (extrusion). The non-incorporated TFL, as it is insoluble in aqueous solutions, crystallises on a needle type structure and remains at the top of the filters. After extrusion through 0,4 µm filter, the formulations are split in two equal parts. With one of those part extrusion procedure continues, now through diameter pore membranes of 0,2 and 0,1 µm, with two passages in the last filter. The liposomal formulation half part that was extruded until membranes of 0,4 µm pore diameter, is named VET400 (Vesicles Extruded Through 400 nm). The liposomal formulation half part that was extruded until membranes of 0,1 µm pore diameter, is named VET400 (Vesicles Extruded Through 100 nm). The VET400 and VET100 formulations obtained by the previous process are finally submitted to dosage (final TFL and final LIP).
**Table 1a** represents the lipid composition effect on the incorporation parameters of liposomes sized until 0,4 µm pore filters. **Table 1b** represents the lipid composition effect on the incorporation parameters of liposomes sized until 0,1 µm pore filters. The formulations were prepared with different lipid compositions, with lipid and TFL quantities presented in the referred tables. Incorporation efficiency (I.E.) represent the ratio between the final (drug to lipid ratio) and the initial (drug to lipid ratio) and is expressed as a percentage. The recovery is also expressed as a percentage and can be referred to drug (TFLf/TFLi) or to lipid (LIPf/LIPi).

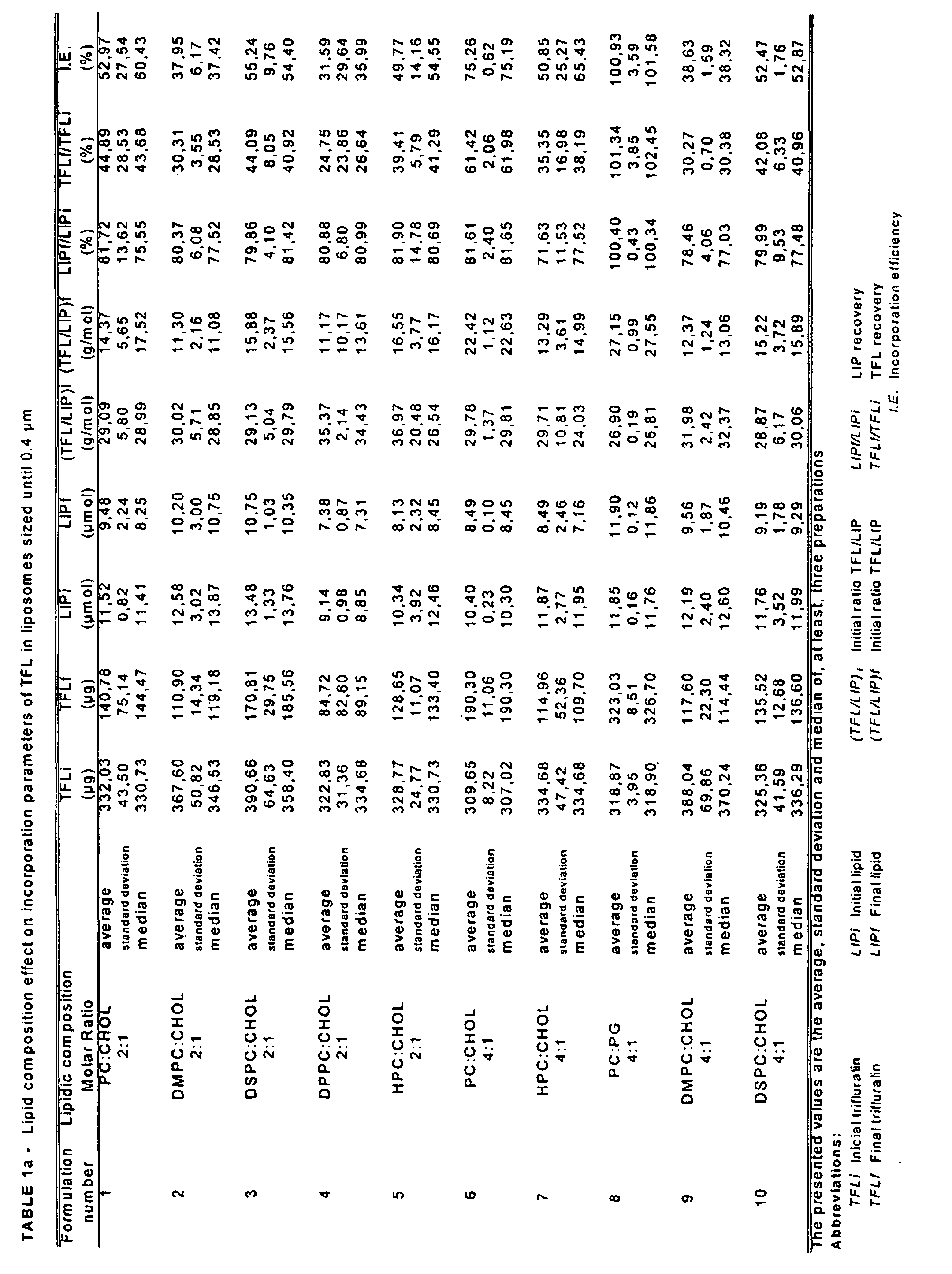

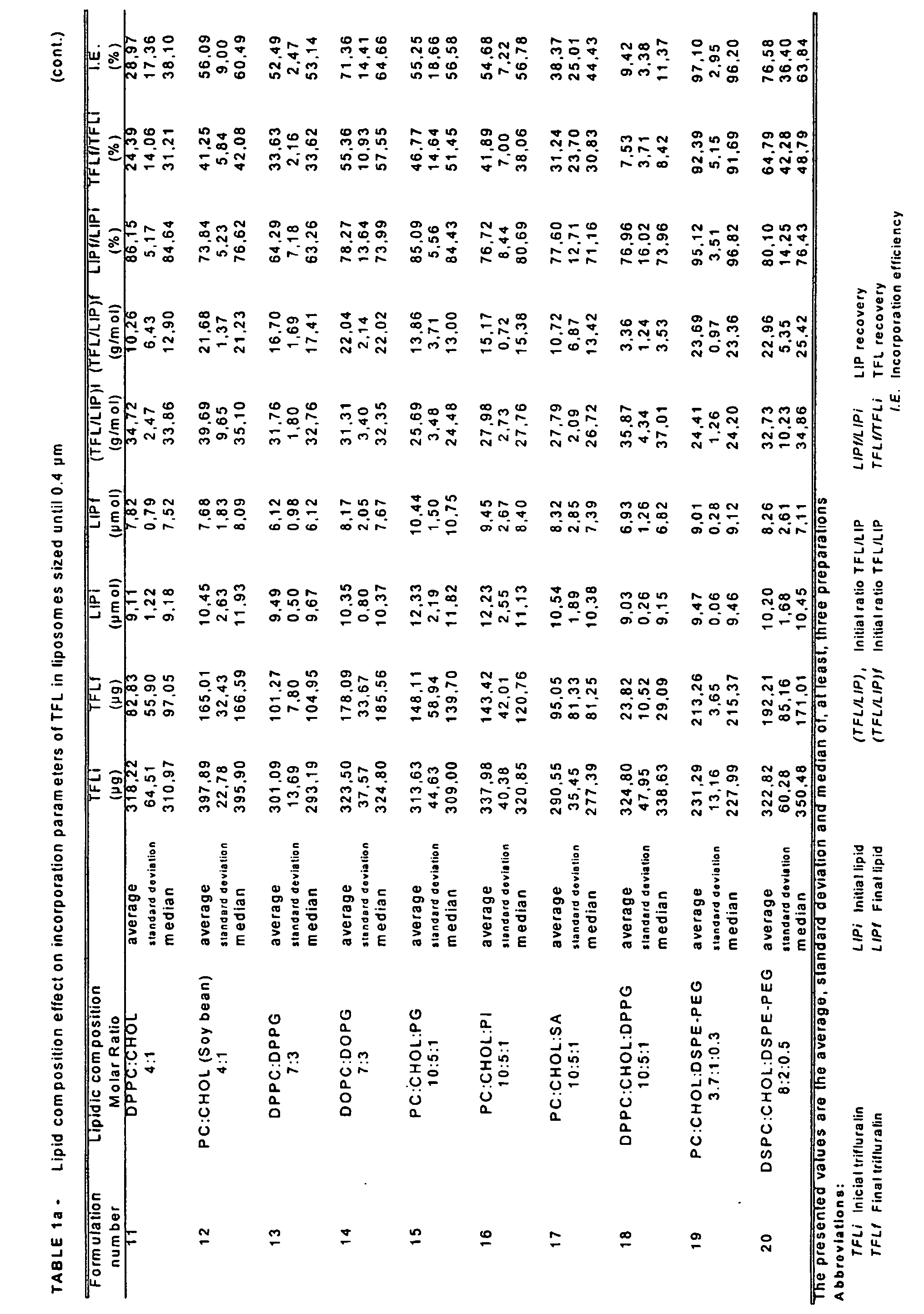

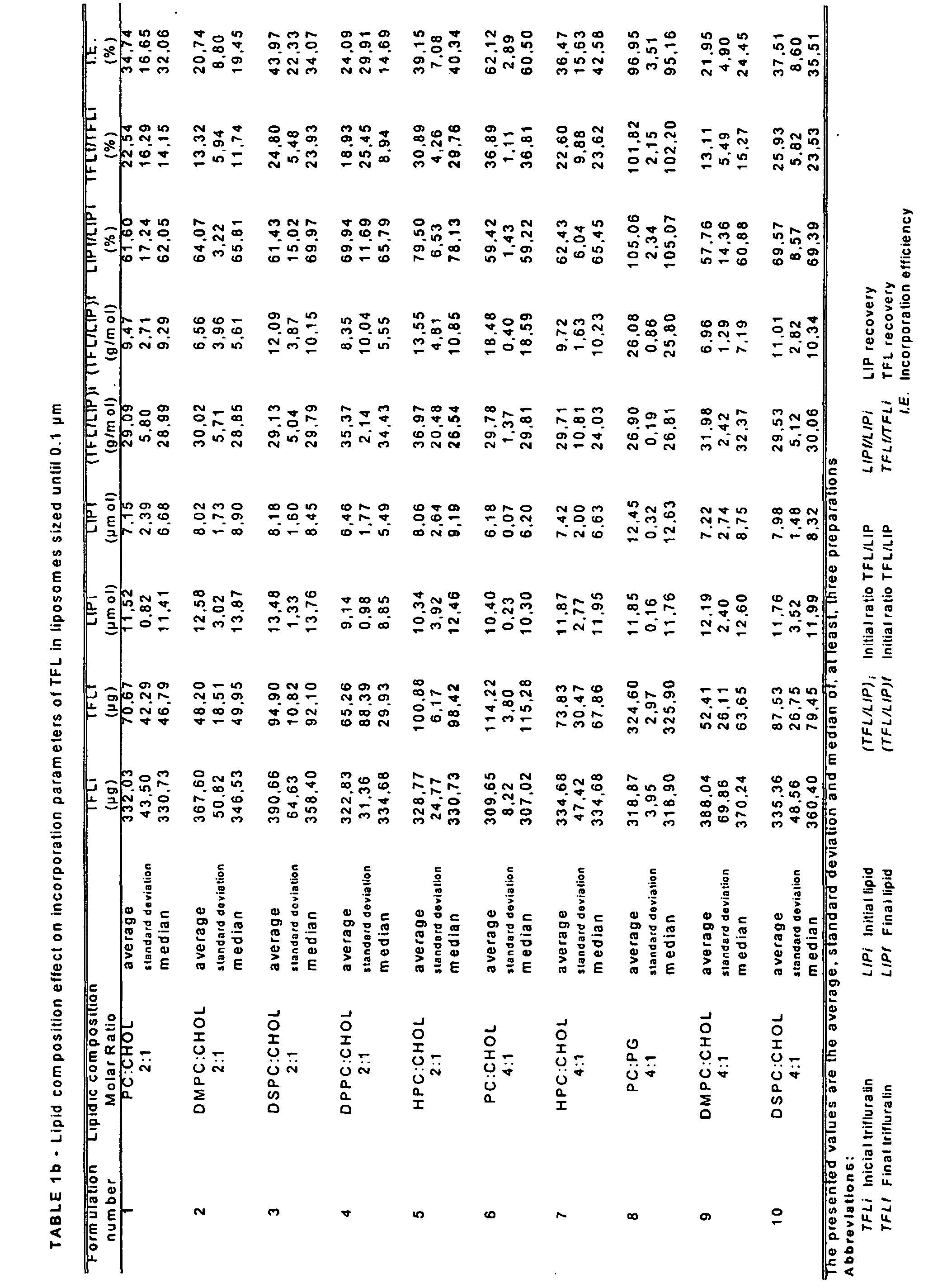

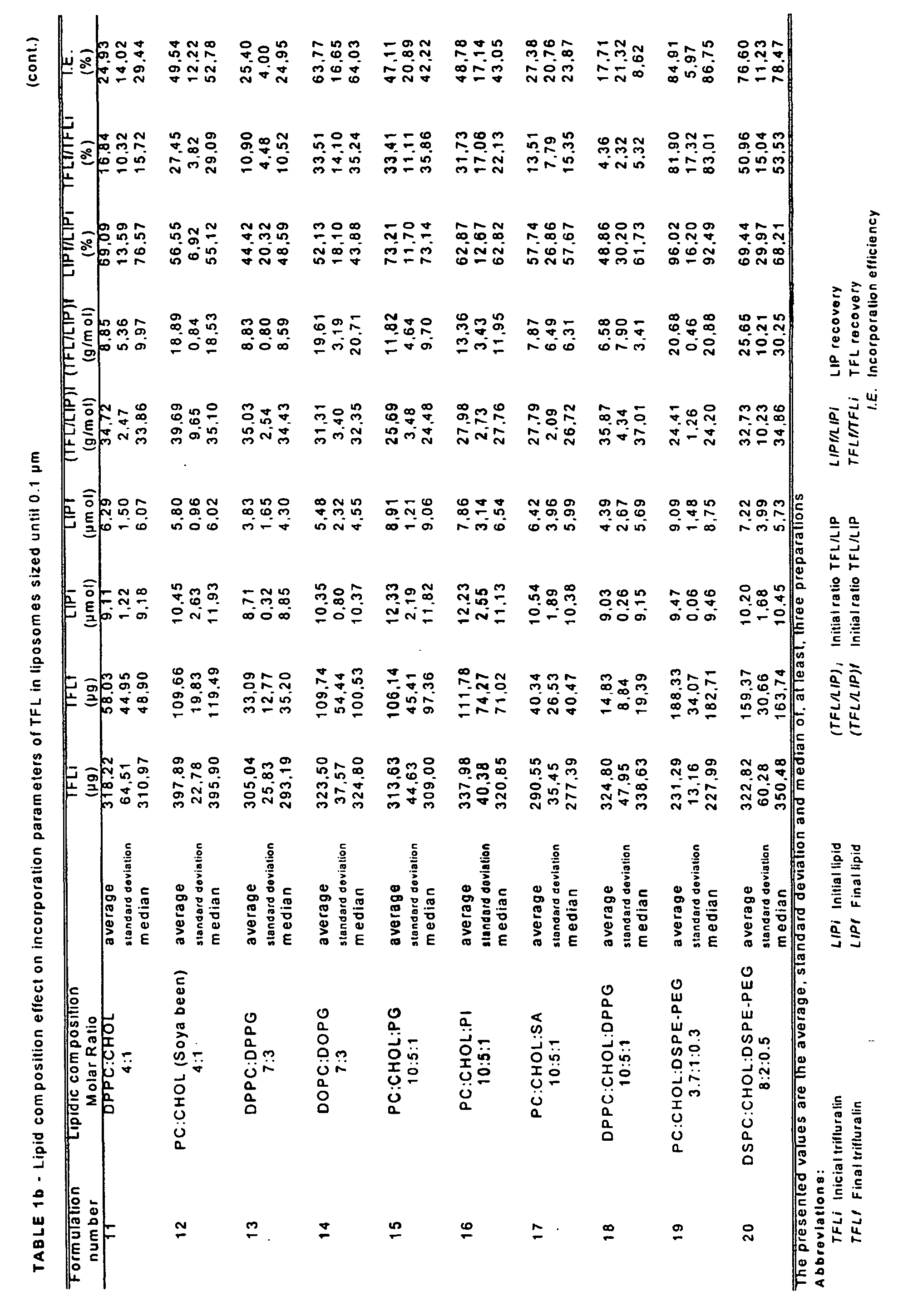

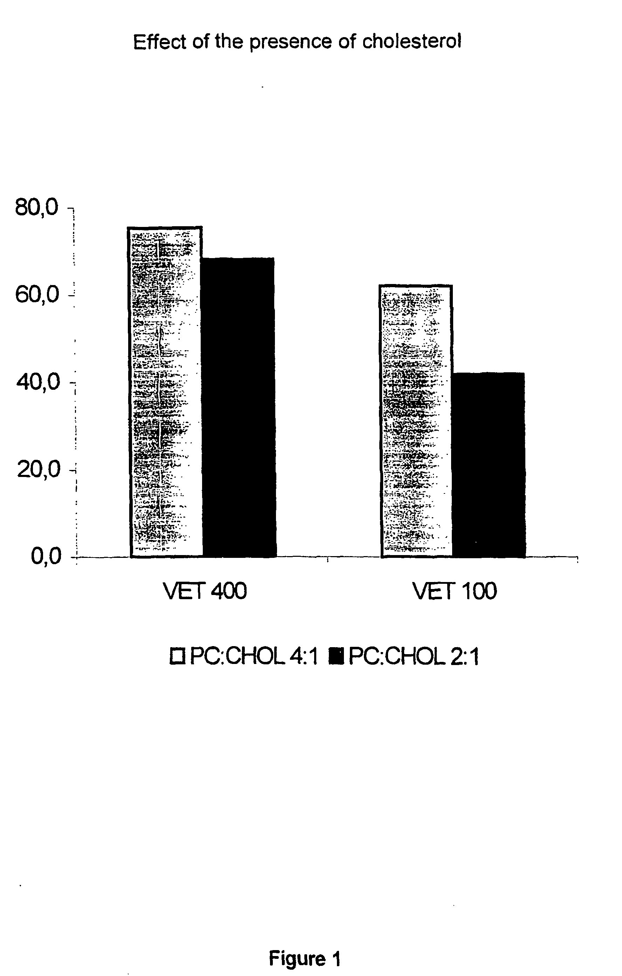

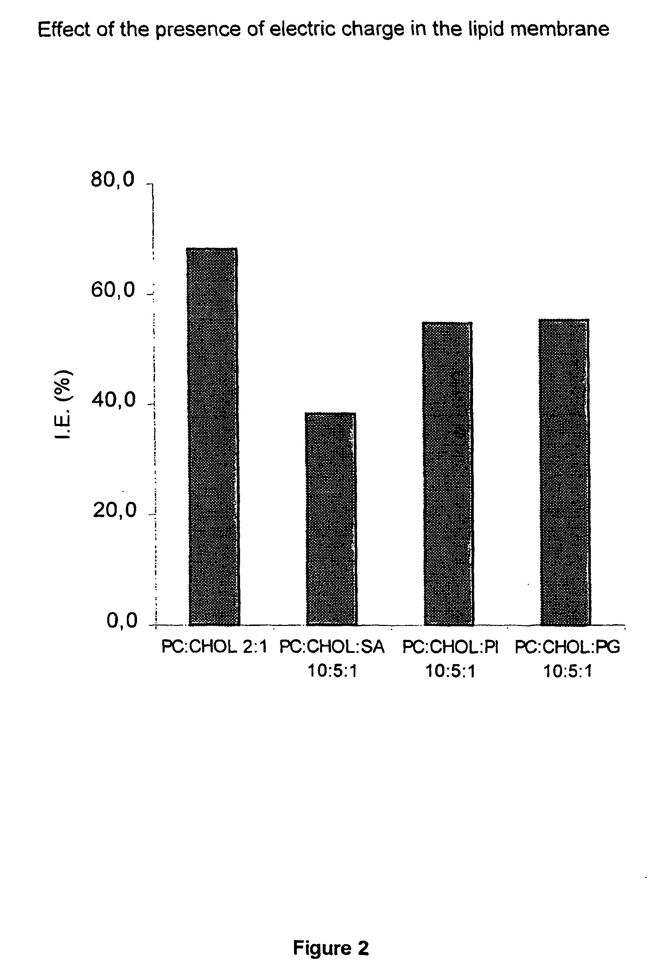

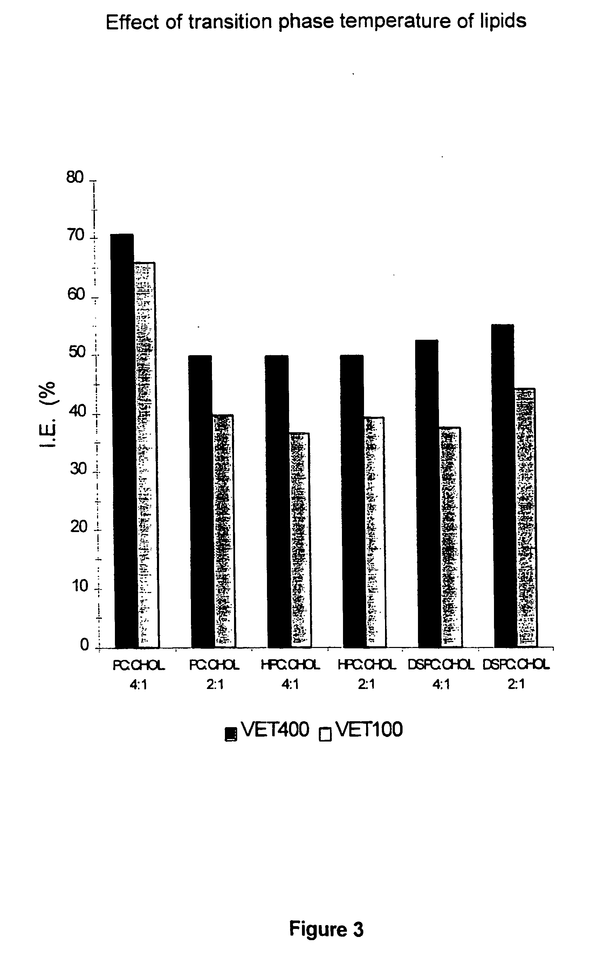

From the analysis of **Table 1a** and **1b** important conclusion can be drawn, referring to the effect of the presence of cholesterol in lipid composition, the effect of electrically charged molecules in lipid composition and to the effect of lipids with different phase transition temperature. The obtained results evidence that TFL is better incorporated in liposomes with low content of cholesterol, as can be observed in **Figure 1** (in the formulation composed of PC:CHOL in a molar ratio of 4:1). The most significative difference was observed to the small size liposomes (VET100). In what concerns the presence of electrically charged molecules in the lipid composition it can be concluded that TFL is poorly incorporated in positively charged liposomes (PC:CHOL:SA) and that, in spite of the better results had been obtained with electrically neutral liposomes (PC:CHOL), negatively charged liposomes (PC:CHOL:PI and PC:CHOL:PG) present good values of incorporation, as can be seen in **Figure 2.** This is an important result, as it is known that negatively charged liposomes, after parenterical administration, have longer circulating times. In what concerns the use of lipids with different phase transition temperature, a comparison can be made between the results obtained for the liposomal formulations with PC, HPC and DSPC (increasing phase transition temperature) in different proportions with CHOL. For the formulations with lower proportion of cholesterol, the lipid with lower transition phase temperature (PC) revealed the best results for TFL incorporation. However, when the cholesterol proportion increases, the lipid with bigger transition phase temperature (DSPC) revealed the best results, even though the difference is not significative, as can be observed in **Figure 3.**

### In vitro stability of two trifluralin liposomal formulations

The preparation of the liposomal formulations for this stability study *in vitro,* 4 mL initial volume containing 10 µmole/mL of lipid (PC:PG) in 4:1 molar ratio and 1 µmole/mL (335 µg/mL) of TFL, started by the addition of TFL to the lipid in chloroform, followed by evaporation of the solvent under nitrogen stream. The hydration of the resulting film was carried out by addition of 400 µL of 0,3 M trehalose, stirring, 15 minute rest, addition of 400 µL more of 0,3 M trehalose, stirring again and resting again for more 15 minute and, finally, with the addition of 3200 µL of 0,3 M trehalose.

The so obtained liposomal formulations were sized by successive filtration under nitrogen pressure, through polycarbonate filters with pores of 5,0, 2,0, 1,0, 0,8, 0,6 and 0,4 µm, with two passages in this late filter (extrusion). The non-incorporated TFL, as it is insoluble in aqueous solutions, crystallises on a needle type structure and remains at the top of the filters. After extrusion through 0,4 µm filter, the formulations are split in two equal parts. With one of those parts extrusion procedure continues, now through diameter pore membranes of 0,2 and 0,1 µm, with two passages in the last filter. The two formulations obtained according to the previous process were kept at 4°C and samples for dosage of TFL were removed at days 0, 1, 2, 3, 4, 6, 8 and 10, being the result expressed by comparison with the value obtained for day O, in percentage. Immediately before the sampling, the formulations were microscopically observed for crystal detection that, if present, would be removed by centrifugation.

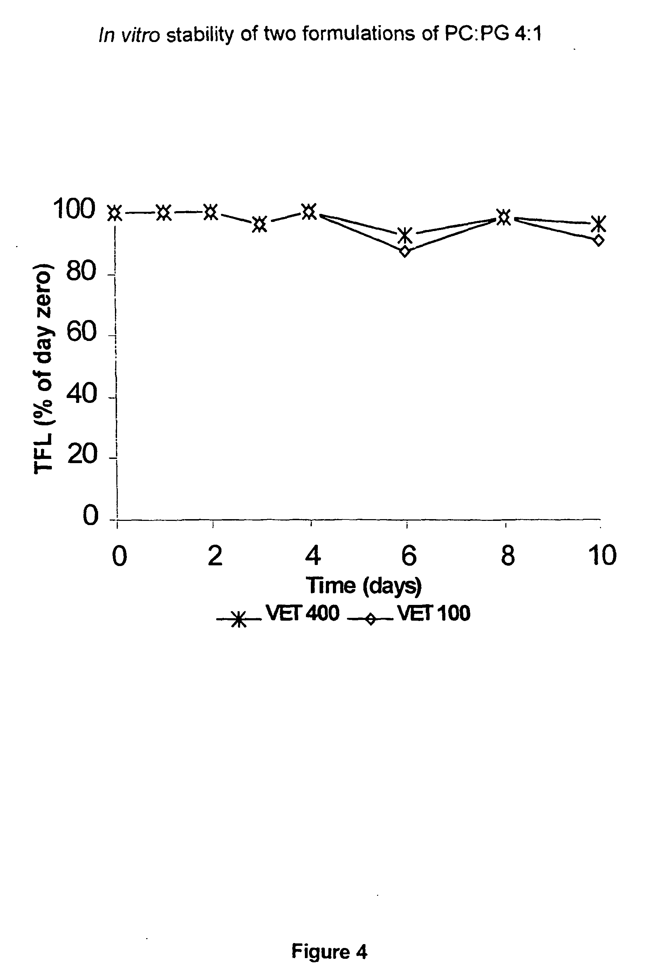

As can be seen in **Figure 4**, the two liposomal formulations (VET 400 and VET100) are stable, upon hydration, presenting 100% stability in the first 48 hours. The experience was repeated three times, being the presented values, the median of the results obtained for each time point.

### Stability on storage of three different trifluralin liposomal formulations

The preparation of the liposomal formulations for this stability study *in vitro*, 45 mL initial volume containing 10 µmole/mL of lipid (DOPC:DOPG) in 7:3 molar ratio and 1 µmole/mL (335 µg/mL) of TFL, started by the addition of TFL to the lipid in chloroform, followed by evaporation of the solvent under nitrogen stream. The hydration of the resulting film was carried out by addition of 4,5 mL of 0,3 M trehalose, stirring, 30 minute rest, addition of 4,5 mL more of 0,3 M trehalose, stirring again and resting again for more 15 minute and, finally, with the addition of 36 mL of 0,3 M trehalose.

The so obtained liposomal formulations were sized by successive filtration under nitrogen pressure, through polycarbonate filters with pores of 5,0, 2,0, 1,0, 0,8, 0,6 and 0,4 µm, with two passages in this late filter (extrusion). The non-incorporated TFL, as it is insoluble in aqueous solutions, crystallises on a needle type structure and remains at the top of the filters. After extrusion through 0,4 µm filter, the formulations are split in two equal parts. With one of those parts extrusion procedure continues, now through diameter pore membranes of 0,2 and 0,1 µm, with two passages in the last filter. From the two formulations VET400 and VET 100 obtained according to the previous process, equal amounts were taken and mixed, being this mixture of the two previous formulations named as MIX liposomal formulation. The three liposomal formulations were then split by vials of 1 mL each, frozen at -70°C during 1 hour and lyophilised overnight. After the lyophilization procedure the vials were closed, under vacuum, sealed with aluminium caps and placed in the benchtop for the entire time of the stability study. For each experimental point (0, 0,03, 0,07, 0,13, 0,23, 0,47, 0,7, 1, 2, 3, 4, 5, 6 and 12 months), vials were opened, hydrated with deionised sterile water until the final volume of 1 mL. The vials containing the liposomal formulations were left to stand for 2 hours. The formulations were microscopically observed for crystal detection that, if present, would be removed by centrifugation. Quantification of TFL was carried out for each formulation and the results expressed as the percentage of TFL as compared to day 0 (final day of lyophilization).

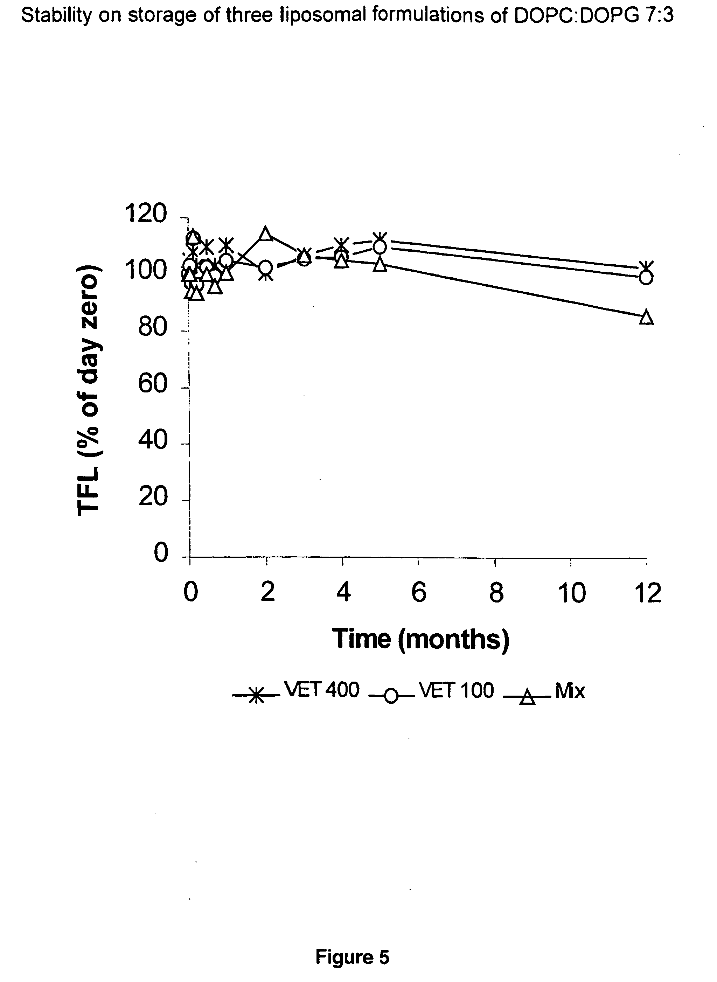

As can be seen from Figure 5, the three liposomal formulations (VET 400, VET100 and MIX) are stable, in the lyophilised form, presenting, after one year of preparation followed by water hydration, TFL values bigger than 90% of the initial value. The experiment was conducted in triplicate and the presented values represent the median of the obtained values for each point.

### Single dose toxicity evaluation

This study was performed with a liposomal formulation of TFL with DOPC:DOPG 7:3 as the lipid composition and compared with a liposomal formulation with equal lipid composition without TFL (empty liposomes).

The preparation of the empty liposomal formulation for this single dose toxicity study, 400 mL initial volume containing 10 µmole/mL of lipid (DOPC:DOPG) in 7:3 molar ratio, started by measuring of lipid in chloroform, followed by evaporation of the solvent under nitrogen stream. The hydration of the resulting film was carried out by addition of 40 mL of 0,3 M sucrose, stirring, 30 minute rest, addition of 40 mL more of 0,3 M sucrose, stirring again and resting again for more 15 minute and, finally, with the addition of 320 mL of 0,3 M sucrose.

The so obtained empty liposomal formulation was sized by successive filtration under nitrogen pressure, through polycarbonate filters with pores of 5,0, 2,0, 1,0, 0,8, 0,6 and 0,4 µm, with two passages in this late filter (extrusion). After extrusion the liposomal formulation was submitted to ultracentrifugation at 49.000 rpm, for two hours, at 15°C. After ultracentrifugation, supernatant was removed and the pellet was ressuspended until 35 mL by addition of 0,3 M sucrose.

The preparation of the TFL liposomal formulation was performed according to the same process described for the empty formulation, with TFL being added to the initial solution of lipid in chloroform. After extrusion through 0,4 µm filter, the formulations are split in two equal parts. With one of those parts extrusion procedure continues, now through diameter pore membranes of 0,2 and 0,1 µm, with two passages in the last filter. The TFL formulations VET400 and VET100 obtained according to the previous process were mixed, being this mixture of the two previous formulations named as MIX liposomal formulation. The formulation was microscopically observed for crystal detection that, if present, would be removed by centrifugation.

The final lipid concentration was determined in both empty and TFL liposomal formulations being the late one adjusted in a way that both formulations contained exactly the same concentration of lipid. After this adjustment the TFL concentration was determined in the TFL containing formulation.

The study was carried out in BALB/c male and female mice. The liposomal formulations were administered by two routes: intraperitoneal (i.p.) and intravenous (i.v.). The administered doses were 30, 20 and 10 mL/kg for the i.p. route and of 10, 5 and 2 mL/kg for the i.v. route of administration. 5 animal per group were used. The administered doses correspond to calculated doses of lipid (in both formulations) and of TFL (in the TFL containing formulation) presented in **Table 2**.

**Table 2 -**

| Single dose toxicity doses | | |
|---|---|---|
| Dose (mL/Kg) | Lipid (µmole/Kg) | TFL (mg/Kg) |
| 30 | 2790 | 63.9 |
| 20 | 1860 | 42.6 |
| 10 | 930 | 21.3 |
| 5 | 465 | 10.7 |
| 2 | 186 | 4.3 |

All animals were weighted and the amount of liposomal formulation was calculated according to the measured weight in order to achieve the desired dose, in mUkg. One animal group per sex was injected with 0,3 M sucrose as control group

The animals were observed at regular intervals, during 48 hours after administration, for detection of behaviour changes. After that period, animals were euthanised, weighted and, from each animal, heart, spleen, liver and kidneys were removed, weighted and observed for macroscopical changes. Relative organ weight was calculated as the ratio between the organ weight and the weight of the animal. The obtained results are presented from **Table 3** to **20.**

**Table 3 -**

| Absolute animal weight (empty liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 3,91E+01 | 2,93E+01 | 3,69E+01 | 2,78E+01 |
| | standard deviation | 3,67E+00 | 1,61E+00 | 1,94E+00 | 1,02E+00 |
| 2 | average | 3,75E+01 | 2,92E+01 | | |
| | standard deviation | 1,42E+00 | 1,99E+00 | | |
| 5 | average | 3,80E+01 | 2,76E+01 | | |
| | standard deviation | 2,73E+00 | 8,12E-01 | | |
| 10 | average | 3,67E+01 | 2,92E+01 | 3,75E+01 | 2,73E+01 |
| | standard deviation | 9,15E-01 | 1,73E+00 | 1,45E+00 | 1,33E+00 |
| 20 | average | | | 3,55E+01 | 2,92E+01 |
| | standard deviation | | | 4,60E+00 | 2,91E+00 |
| 30 | average | | | 3,48E+01 | 2,94E+01 |
| | standard deviation | | | 1,52E+00 | 1,60E+00 |

**Table 4 -**

| Absolute heart weight (empty liposome) | | | | | |
|---|---|---|---|---|---|
| Dose (mL/Kg) | | *Administration routes* | | | |
| | | i.v. | | i.p. | |
| | | Males | Females | Males | Females |
| Control | average | 1,70E-01 | 1,42E-01 | 2,07E-01 | 1,44E-01 |
| | standard deviation | 2,55E-02 | 5,20E-03 | 2,38E-02 | 2,13E-02 |
| 2 | average | 1,87E-01 | 1,55E-01 | | |
| | standard deviation | 8,81E-03 | 1,67E-02 | | |
| 5 | average | 1,85E-01 | 1,37E-01 | | |
| | standard deviation | 1,95E-02 | 2,09E-02 | | |
| 10 | average | 1,96E-01 | 1,50E-01 | 2,11E-01 | 1,33E-01 |
| | standard deviation | 3,68E-02 | 6,17E-03 | 3,63E-02 | 2,15E-02 |
| 20 | average | | | 2,15E-01 | 1,34E-01 |
| | standard deviation | | | 3,04E-02 | 1,07E-02 |
| 30 | average | | | 1,92E-01 | 1,49E-01 |
| | standard deviation | | | 1,18E-02 | 2,47E-02 |

**Table 5 -**

| Absolute liver weight (empty liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 1,72E+00 | 1,31E+00 | 1,89E+00 | 1,07E+00 |
| | standard deviation | 2,08E-01 | 4,25E-02 | 2,07E-01 | 9,86E-02 |
| 2 | average | 1,72E+00 | 1,24E+00 | | |
| | standard deviation | 7,32E-02 | 1,39E-01 | | |
| 5 | average | 1,62E+00 | 1,12E+00 | | |
| | standard deviation | 1,74E-01 | 8,91E-02 | | |
| 10 | average | 1,68E+00 | 1,21E+00 | 1,96E+00 | 1,06E+00 |
| | standard deviation | 8,40E-02 | 1,40E-01 | 1,14E-01 | 7,88E-02 |
| 20 | average | | | 1,95E+00 | 1,20E+00 |
| | standard deviation | | | 1,12E-01 | 1,68E-01 |
| 30 | average | | | 1,58E+00 | 1,19E+00 |
| | standard deviation | | | 1,25E-01 | 1,40E-01 |

**Table 6 -**

| Absolute spleen weight (empty liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 1,03E-01 | 9,46E-02 | 1,26E-01 | 1,04E-01 |
| | standard deviation | 1,08E-02 | 5,36E-02 | 2,51E-02 | 2,66E-02 |
| 2 | average | 1,10E-01 | 9,86E-02 | | |
| | standard deviation | 1,51 E-02 | 4,86E-02 | | |
| 5 | average | 1,21E-01 | 7,60E-02 | | |
| | standard deviation | 9,11E-03 | 2,53E-02 | | |
| 10 | average | 1,16E-01 | 1,07E-01 | 1,06E-01 | 7,46E-02 |
| | standard deviation | 1,14E-02 | 2,55E-02 | 8,21E-03 | 2,36E-02 |
| 20 | average | | | 1,27E-01 | 1,01E-01 |
| | standard deviation | | | 1,88E-02 | 1,88E-02 |
| 30 | average | | | 1,08E-01 | 8,97E-02 |
| | standard deviation | | | 8,35E-03 | 1,86E-02 |

**Table 7 -**

| Absolute kidneys weight (empty liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 6,37E-01 | 3,64E-01 | 6,22E-01 | 3,11 E-01 |
| | standard deviation | 7,83E-02 | 2,62E-02 | 5,57E-02 | 4,00E-02 |
| 2 | average | 6,13E-01 | 3,63E-01 | | |
| | standard deviation | 6,42E-02 | 2,76E-02 | | |
| 5 | average | 6,46E-01 | 3,20E-01 | | |
| | standard deviation | 1,11 E-01 | 4,23E-02 | | |
| 10 | average | 6,63E-01 | 3,99E-01 | 6,02E-01 | 2,95E-01 |
| | standard deviation | 6,17E-02 | 4,82E-02 | 6,61E-02 | 2,20E-02 |
| 20 | average | | | 5,24E-01 | 3,00E-01 |
| | standard deviation | | | 2,62E-01 | 1,80E-02 |
| 30 | average | | | 6,29E-01 | 3,17E-01 |
| | standard deviation | | | 6,37E-02 | 2,64E-02 |

**Table 8 -**

| Absolute animal weight (TFL containing liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 2,61E+01 | 2,06E+01 | 2,64E+01 | 1,96E+01 |
| | standard deviation | 1,42E+00 | 1,13E+00 | 1,82E+00 | 1,93E+00 |
| 2 | average | 2,52E+01 | 1,89E+01 | | |
| | standard deviation | 7,53E-01 | 1,28E+00 | | |
| 5 | average | 2,58E+01 | 1,97E+01 | | |
| | standard deviation | 1,99E+00 | 7,31 E-01 | | |
| 10 | average | 2,62E+01 | 2,01E+01 | 2,45E+01 | 2,05E+01 |
| | standard deviation | 1,32E+00 | 9,32E-01 | 1,79E+00 | 1,60E+00 |
| 20 | average | | | 2,71E+01 | 2,07E+01 |
| | standard deviation | | | 2,32E+00 | 6,96E-01 |
| 30 | average | | | 1,96E+01 | 2,02E+01 |
| | standard deviation | | | 1,93E+00 | 2,67E+00 |

**Table 9 -**

| Absolute heart weight (TFL containing liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 1,43E-01 | 1,08E-01 | 1,38E-01 | 9,60E-02 |
| | standard deviation | 1,28E-02 | 1,10E-02 | 1,92E-02 | 8,98E-03 |
| 2 | average | 1,39E-01 | 1,05E-01 | | |
| | standard deviation | 9,49E-03 | 1,18E-02 | | |
| 5 | average | 1,37E-01 | 1,14E-01 | | |
| | standard deviation | 7,03E-03 | 8,61E-03 | | |
| 10 | average | 1,54E-01 | 1,04E-01 | 1,30E-01 | 9,65E-02 |
| | standard deviation | 9,80E-03 | 1,48E-02 | 1,56E-02 | 9,46E-03 |
| 20 | average | | | 1,57E-01 | 1,05E-01 |
| | standard deviation | | | 3,50E-02 | 5,74E-03 |
| 30 | average | | | 1,34E-01 | 9,62E-02 |
| | standard deviation | | | 1,55E-02 | 1,17E-02 |

**Table 10 -**

| Absolute liver weight (TFL containing liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 1,32E+00 | 9,71E-01 | 1,19E+00 | 8,80E-01 |
| | standard deviation | 5,16E-02 | 8,62E-02 | 7,82E-02 | 1.10E-01 |
| 2 | average | 1,20E+00 | 9,63E-01 | | |
| | standard deviation | 1,21E-01 | 8,88E-02 | | |
| 5 | average | 1,31E+00 | 9,79E-01 | | |
| | standard deviation | 1,76E-01 | 2,86E-02 | | |
| 10 | average | 1,17E+00 | 1,00E+00 | 1,21E+00 | 1,01E+00 |
| | standard deviation | 6,32E-01 | 5,03E-02 | 1,40E-01 | 8,45E-02 |
| 20 | average | | | 1,40E+00 | 9,97E-01 |
| | standard deviation | | | 1,30E-01 | 6,59E-02 |
| 30 | average | | | 1,27E+00 | 9,19E-01 |
| | standard deviation | | | 1,48E-01 | 1,73E-01 |

**Table 11 -**

| Absolute spleen weight (TFL containing liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 9,02E-02 | 8,52E-02 | 8,25E-02 | 7,66E-02 |
| | standard deviation | 5,55E-03 | 8,24E-03 | 3,97E-03 | 1,75E-02 |
| 2 | average | 8,65E-02 | 7,98E-02 | | |
| | standard deviation | 1,28E-02 | 8,21 E-03 | | |
| 5 | average | 9,47E-02 | 9,41E-02 | | |
| | standard deviation | 2,51 E-02 | 4,85E-03 | | |
| 10 | average | 1,02E-01 | 9,16E-02 | 9,52E-02 | 9,05E-02 |
| | standard deviation | 2,24E-02 | 6,22E-03 | 7,56E-03 | 8,78E-03 |
| 20 | average | | | 9,45E-02 | 9,65E-02 |
| | standard deviation | | | 8,13E-03 | 5,22E-03 |
| 30 | average | | | 1,10E-01 | 8,72E-02 |
| | standard deviation | | | 4,03E-02 | 1,58E-02 |

**Table 12 -**

| Absolute kidneys weight (TFL containing liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 3,86E-01 | 2,32E-01 | 3,56E-01 | 2,21E-01 |
| | standard deviation | 3,53E-02 | 2,41 E-02 | 2,80E-02 | 3,16E-02 |
| 2 | average | 3,52E-01 | 2,13E-01 | | |
| | standard deviation | 1,30E-02 | 1,98E-02 | | |
| 5 | average | 3,62E-01 | 2,15E-01 | | |
| | standard deviation | 4,37E-02 | 4,90E-02 | | |
| 10 | average | 3,90E-01 | 2,25E-01 | 3,36E-01 | 2,17E-01 |
| | standard deviation | 2,35E-02 | 1,92E-02 | 3,23E-02 | 2,46E-02 |
| 20 | average | | | 3,61E-01 | 2,38E-01 |
| | standard deviation | | | 4,23E-02 | 1,88E-02 |
| 30 | average | | | 3,48E-01 | 2,14E-01 |
| | standard deviation | | | 2,94E-02 | 1,76E-02 |

**Table 13 -**

| Relative heart weight (empty liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 4,35E-03 | 4,88E-03 | 5,61 E-03 | 5,18E-03 |
| | standard deviation | 4,87E-04 | 3,60E-04 | 7,34E-04 | 7,66E-04 |
| 2 | average | 4,99E-03 | 5,31E-03 | | |
| | standard deviation | 1,62E-04 | 2,77E-04 | | |
| 5 | average | 4,88E-03 | 4,96E-03 | | |
| | standard deviation | 5,63E-04 | 8,06E-04 | | |
| 10 | average | 4,88E-03 | 5,16E-03 | 5,61E-03 | 4,91E-03 |
| | standard deviation | 3,60E-04 | 3,89E-04 | 9,03E-04 | 8,90E-04 |
| 20 | average | | | 6,08E-03 | 4,61 E-03 |
| | standard deviation | | | 8,16E-04 | 4,65E-04 |
| 30 | average | | | 5,52E-03 | 5,07E-03 |
| | standard deviation | | | 2,82E-04 | 8,75E-04 |

**Table 14 -**

| Relative liver weight (empty liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 4,39E-02 | 4,48E-02 | 5,12E-02 | 3,87E-02 |
| | standard deviation | 1,45E-03 | 2,38E-03 | 4,75E-03 | 3,30E-03 |
| 2 | average | 4,58E-02 | 4,27E-02 | | |
| | standard deviation | 2,02E-03 | 2,84E-03 | | |
| 5 | average | 4,27E-02 | 4,07E-02 | | |
| | standard deviation | 3,10E-03 | 2,43E-03 | | |
| 10 | average | 4,58E-02 | 4,14E-02 | 5,24E-02 | 3,86E-02 |
| | standard deviation | 2,98E-03 | 3,03E-03 | 3,36E-03 | 1,43E-03 |
| 20 | average | | | 5,59E-02 | 4,09E-02 |
| | standard deviation | | | 9,62E-03 | 3,32E-03 |
| 30 | average | | | 4,53E-02 | 4,04E-02 |
| | standard deviation | | | 2,71E-03 | 4,22E-03 |

**Table 15 -**

| Relative spleen weight (empty liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 2,65E-03 | 3,29E-03 | 3,42E-03 | 3,75E-03 |
| | standard deviation | 3,51E-04 | 1,95E-03 | 6,65E-04 | 9,22E-04 |
| 2 | average | 2,94E-03 | 3,33E-03 | | |
| | standard deviation | 4,13E-04 | 1,59E-03 | | |
| 5 | average | 3,19E-03 | 2,75E-03 | | |
| | standard deviation | 2,49E-05 | 9,28E-04 | | |
| 10 | average | 3,18E-03 | 3,67E-03 | 2,81E-03 | 2,74E-03 |
| | standard deviation | 3,57E-04 | 7,30E-04 | 1,26E-04 | 8,96E-04 |
| 20 | average | | | 3,66E-03 | 3,46E-03 |
| | standard deviation | | | 9,83E-04 | 4,51 E-04 |
| 30 | average | | | 3,11 E-03 | 3,05E-03 |
| | standard deviation | | | 1,42E-04 | 6,26E-04 |

**Table 16 -**

| Relative kidneys weight (empty liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 1,63E-02 | 1,24E-02 | 1,69E-02 | 1,12E-02 |
| | standard deviation | 1,36E-03 | 4,49E-04 | 1,81E-03 | 1,12E-03 |
| 2 | average | 1,63E-02 | 1,25E-02 | | |
| | standard deviation | 1,27E-03 | 3,47E-04 | | |
| 5 | average | 1,69E-02 | 1,16E-02 | | |
| | standard deviation | 2,22E-03 | 1,64E-03 | | |
| 10 | average | 1,81E-02 | 1,37E-02 | 1,60E-02 | 1,08E-02 |
| | standard deviation | 1,79E-03 | 1,53E-03 | 1,50E-03 | 1,16E-03 |
| 20 | average | | | 1,52E-02 | 1,03E-02 |
| | standard deviation | | | 8,41E-03 | 6,26E-04 |
| 30 | average | | | 1,81E-02 | 1,08E-02 |
| | standard deviation | | | 1,51E-03 | 1,10E-03 |

**Table 17 -**

| Relative heart weight (TFL containing liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 5,49E-03 | 5,27E-03 | 5,25E-03 | 4,90E-03 |
| | standard deviation | 4,52E-04 | 5,20E-04 | 7,39E-04 | 1,97E-04 |
| 2 | average | 5,50E-03 | 5,56E-03 | | |
| | standard deviation | 4,97E-04 | 7,85E-04 | | |
| 5 | average | 5,35E-03 | 5,79E-03 | | |
| | standard deviation | 5,73E-04 | 5,42E-04 | | |
| 10 | average | 5,90E-03 | 5,20E-03 | 5,28E-03 | 4,70E-03 |
| | standard deviation | 5,87E-04 | 7,05E-04 | 3,02E-04 | 2,23E-04 |
| 20 | average | | | 5,77E-03 | 5,07E-03 |
| | standard deviation | | | 1,04E-03 | 2,44E-04 |
| 30 | average | | | 5,19E-03 | 4,79E-03 |
| | standard deviation | | | 4,46E-04 | 3,87E-04 |

**Table 18 -**

| Relative liver weight (TFL containing liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 5,07E-02 | 4,72E-02 | 4,50E-02 | 4,48E-02 |
| | standard deviation | 3,17E-03 | 3,28E-03 | 2,38E-03 | 1,82E-03 |
| 2 | average | 4,77E-02 | 5,09E-02 | | |
| | standard deviation | 4,03E-03 | 3,25E-03 | | |
| 5 | average | 5,06E-02 | 4,98E-02 | | |
| | standard deviation | 4,96E-03 | 1,78E-03 | | |
| 10 | average | 4,43E-02 | 4,99E-02 | 4,93E-02 | 4,93E-02 |
| | standard deviation | 2,34E-02 | 2,75E-03 | 4,63E-03 | 1,43E-03 |
| 20 | average | | | 5,15E-02 | 4,81 E-02 |
| | standard deviation | | | 1,31E-03 | 1,96E-03 |
| 30 | average | | | 4,93E-02 | 4,53E-02 |
| | standard deviation | | | 3,60E-03 | 2,70E-03 |

**Table 19 -**

| Relative spleen weight (TFL containing liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 3,47E-03 | 4,15E-03 | 3,13E-03 | 3,87E-03 |
| | standard deviation | 2,14E-04 | 3,94E-04 | 1,04E-04 | 6,85E-04 |
| 2 | average | 3,42E-03 | 4,22E-03 | | |
| | standard deviation | 4,35E-04 | 3,83E-04 | | |
| 5 | average | 3,65E-03 | 4,78E-03 | | |
| | standard deviation | 8,45E-04 | 1,91 E-04 | | |
| 10 | average | 3,86E-03 | 4,57E-03 | 3,90E-03 | 4,41 E-03 |
| | standard deviation | 6,87E-04 | 2,34E-04 | 4,63E-04 | 1,18E-04 |
| 20 | average | | | 3,49E-03 | 4,66E-03 |
| | standard deviation | | | 2,25E-04 | 2,21 E-04 |
| 30 | average | | | 4,24E-03 | 4,32E-03 |
| | standard deviation | | | 1,33E-03 | 4,76E-04 |

**Table 20 -**

| Relative kidneys weight (TFL containing liposomes) | | | | | |
|---|---|---|---|---|---|
| Dose (mL/Kg) | | *Administration routes* | | | |
| | | i.v. | | i.p. | |
| | | Males | Females | Males | Females |
| Control | average | 1,48E-02 | 1,13E-02 | 1,35E-02 | 1,12E-02 |
| | standard deviation | 1,28E-03 | 5,42E-04 | 1,03E-03 | 5,59E-04 |
| 2 | average | 1,40E-02 | 1, 12E-02 | | |
| | standard deviation | 8,36E-04 | 5,44E-04 | | |
| 5 | average | 1,40E-02 | 1,09E-02 | | |
| | standard deviation | 1,19E-03 | 2,57E-03 | | |
| 10 | average | 1,49E-02 | 1,12E-02 | 1,37E-02 | 1,06E-02 |
| | standard deviation | 6,29E-04 | 6,70E-04 | 6,60E-04 | 7,15E-04 |
| 20 | average | | | 1,33E-02 | 1,15E-02 |
| | standard deviation | | | 8,20E-04 | 5,48E-04 |
| 30 | average | | | 1,35E-02 | 1,07E-02 |
| | standard deviation | | | 8,32E-04 | 7,50E-04 |

The obtained values were statistically treated for the significance of variations (p=0,005). Obtained results for absolute weights (full animal and separate organs) in both formulations were compared between themselves and with absolute weight of control group. The total absence of toxicity of any of the injected formulations was concluded from the statistical analysis.

### Biological activity evaluation

In order to evaluate the biological activity of TFL liposomal formulations prepared according to the present invention, one animal model of leishmaniasis was selected. BALB/c mice were infected with 2 x 10⁷ (i.v.) LV-9 (*Leishmania donovani*) parasites, obtained from the London School of Hygiene and Tropical Medicine. The groups (5 animals per group) and treatment schedules are presented in **Table 21**.

**Table 21 -**

| Biological activity | | | |
|---|---|---|---|
| Formulation lipidic composition | Dose (mg TFL/kg) | N^{er} of Doses | Inhibition % |
| | | 5 | 62 |
| DOPC:DOPG 7:3 | 15 | 3 | 17 |
| | | 1 | 80 |
| | | 5 | 53 |
| DSPC:CHOL 4:1 | 15 | 3 | 92 |
| | | 1 | 91 |
| | | 5 | 47 |
| PC:PG 4:1 | 15 | 3 | 88 |
| | | 1 | 60 |
| | | 5 | 57 |
| PC:CHOL:PI 3.7:1:0,3 | 5 | 3 | 52 |
| | | 1 | 68 |
| PC:CHOL:DSPE-PEG (2000) 3.7:1:0,3 | 4 | 5 | 75 |
| | | 3 | 74 |
| DOPC:DOPG 7:3 (dialysed) | 0,6 | 5 | 86 |

Treatments started 7 days post-infection and animals were euthanised 15 days post-infection. Liver was removed and weighted from each animal and amastigote counting was performed in each one by smear impression. The infection was calculated through an appropriate mathematical equation. The so obtained results are expressed in **Table 21**.

The first conclusion is that, due to the liposomal incorporation of TFL, parenteric administration of TFL is possible.

All TFL liposomal formulations were able to reduce infection in this model.

## Claims

1. A liposomal formulation containing one dinitroaniline incorporated or encapsulated, for example, trifluralin, for use in the preparation of a pharmaceutical formulation, **characterized by** the fact of containing a mixture of two distinct liposome populations of particles with mean diameters respectively bigger and lower than 100 nm.

2. A liposomal formulation, according to claim 1, **characterized by** the fact that of mixing populations of particles, respectively bigger than 400 nm and lower than 50 nm.

3. A process for the preparation of a liposomal formulation containing a dinitroaniline, composed of two distinct liposome populations of particles with mean diameters respectively bigger and lower than 100 nm, which comprises the following steps:
• obtention of the liposomal formulations containing vesicles of dinitroaniline by hydration, with a solution containing an antisublimating agent of a lipid film containing the dinitroaniline
• obtention of the different populations with well-defined diameters by a sizing step
• mixing the obtained distinct populations;
• lyophilization/dehydration of the so obtained liposomal formulation; and
• rehydration of the dehydrated liposomal formulation

4. Process according to claim 3, **characterized by** the performing of the sizing step of the dinitroaniline liposomal formulation in order to reduce the vesicles diameter, done previously to the dehydration step.

5. Process, according to claim 4, **characterized by** the performing the sizing step by extrusion under pressure through porous membranes.

6. Process, according to any of the claims 3 to 5, **characterized by** the fact that the hydration is carried out by the addition of a small amount of an aqueous solution, followed by the addiction of the remaining volume of the aqueous solution, after a resting period.

7. Process according to claim 6, **characterized by** the fact of performing the rehydration steps with saccharose, trehalose, glucose or any other sugar solution.

8. Process, according to any of the claims 3 to 7, **characterized by** the fact of mixing different diameter particle populations.

9. Process, according to claim 8, **characterized by** the fact of mixing particles that, after sizing, present population with diameters of, respectively, bigger and lower than 100 nm.

10. Process, according to claim 9, **characterized by** the fact of performing the sizing step according to claim 5.

11. Process, according to any of the claims 3 to 5 or 9 to 10, **characterized by** the fact that the hydration is performed according to claim 6.

12. Process, according to any of the claims 3 to 6 or 9 to 11, **characterized by** the fact of using in the hydration step a solution according to claim 7.

13. Process, according to any of the claims 3 to 7 or 9 to 12, **characterized by** the fact of using solutions according to claim 8.

14. Process, according to any of he claims 3 to 13, **characterized by** the use of any of the following lipids, hydrogenated or not, individually or in mixtures, in any molar ratio: distearoylphosphatidylcholine (DSPC), phosphatidylcholine (PC), cholesterol (Chol) or derivatives, sphingomielin (SM), dioleoylphosphatidylcholine (DOPC), dioleoylphosphatidylglycerol (DOPG), phosphatidylglycerol (PG), dimiristoylphosphatidylcholine (DMPC), dipalmitoylphosphatidylcholine (DPPC), gangliosides, ceramides, phosphatidylinositol (PI), phosphatydic acid, (PA), dicetylphosphate (DcP), dimiristoylphosphatidylglycerol (DMPG), stearylamine (SA) dipalmitoylphosphatidylglycerol (DPPG) and other synthethic lipids.

15. Process, according to any of the claims 3 to 14, **characterized by** the fact of the dinitroaniline is trifluralin.

16. Liposomal formulations according to any of the claims 1 and 2, when prepared by a process according to any of the claims 3 to 15.

17. Use of the liposomal formulations for the preparation of a pharmaceutical formulation for the treatment in humans or animals, **characterized by** the use of a therapeutic quantity of a dinitroaniline liposomal formulation according to any of the claims 1, 2 and 16.

## Patentansprüche

1. Liposomale Formulierung enthaltend eine umgehülte oder verkapselte Dinitroanilin, zum Beispiel Trifluralin, für Verwendung zur Herstellung einer pharmazeutische Formulierung, **dadurch gekennzeichnet, daß** die liposomale Formulierung eine Mischung aus zwei verschiedenen liposomalen Partikelpopulationen jeweils mit einen durchschnittlichen Durchmesser großer bzw. niedriger als 100 nm enthält.

2. Liposomale Formulierung nach Anspruch 1, **dadurch gekennzeichnet, daß** die Partikelpopulationen, jeweils großer als 400 nm bzw. nidrieger als 50 nm gemischt werden.

3. Verfahren zur Herstellung einer liposomale Formulierung enthaltend eines Dinitroanilin aus zwei verschiedenen liposomalen Partikelpopulationen, jeweils mit einen durchschnittlichen Durchmesser großer bzw. niedriger als 100 nm, das die nachfolgenden Schritten umfasst:
• Gewinnung der liposomalen Formulierungen enthaltend Dinitroanilinbläschen durch Hydratation mit einer Lösung enthaltend einer Antisublimierungsmittel aus einem Lipidschicht daß das Dinitroanilin enthaltet
• Gewinnung von verschiedenen Populationen mit genauen Durchmesser durch eine Kalibrierungsschritt
• Vermischen der erhalteten verschiedenen Populationen;
• Gefriertrocknung/Dehydratation der so erhalteten liposomale Formulierung; und
• Wiederhydratation der dehydratierte liposomale Formulierung

4. Verfahren nach Anspruch 3, **dadurch gekennzeichnet, daß** bevor der Dehydratationsschritt durchgeführt wird, Kalibriert man die liposomale Formulierung aus Dinitroanilin, um den Bläschendurchmesser zu verkleinern.

5. Verfahren nach Anspruch 4, **dadurch gekennzeichnet, daß** die Kalibrierungsschritt durch Extrusion unter Druck durch porigen Membranen durchgeführt wird.

6. Verfahren nach einem oder mehreren der Ansprüchen 3 bis 5, **dadurch gekennzeichnet, daß** die Hydratation durch Zugabe von eine kleine Menge einer wässrige Lösung durchgeführt wird, folgt bei die Zugabe der restliche Menge der wassrige Lösung nach einer Ruhezeit.

7. Verfahren nach Anspruch 6, **dadurch gekennzeichnet, daß** die Wiederhydratationsschritt mit Saccharose-, Trehalose-, Glucose- oder irgendeiner Zuckerlösung durchgeführt wird.

8. Verfahren nach einem oder mehreren der Ansprüchen 3 bis 7, **dadurch gekennzeichnet, daß** Partikelpopulationen mit verschiedenen Durchmesser gemischt werden.

9. Verfahren nach Anspruch 8, **dadurch gekennzeichnet, daß** die Partikeln gemischt werden und, nach diekalibrierung entsteht, eine Population mit Durchmesser großer bzw. niedriger als 100 nm aufweist.

10. Verfahren nach Anspruch 9, **dadurch gekennzeichnet, daß** die Kalibrierungsschritt gemäß Anspruch 5 durchgeführt wird.

11. Verfahren nach einem oder mehreren der Ansprüchen 3 bis 5 oder 9 bis 10, **dadurch gekennzeichnet, daß** die Hydratation gemäß Anspruch 6 durchgeführt wird.

12. Verfahren nach einem oder mehreren der Ansprüchen 3 bis 6 oder 9 bis 11, **dadurch gekennzeichnet, daß** bei der Hydratationsschitt eine Lösung gemäß Anspruch 7 eingesetzt wird.

13. Verfahren nach einem oder mehreren der Ansprüchen 3 bis 7 oder 9 bis 12, **dadurch gekennzeichnet, daß** Lösungen gemäß Anspruch 8 eingesetzt werden.

14. Verfahren nach einem oder mehreren der Ansprüchen 3 bis 13, **dadurch gekennzeichnet, daß** beliebiger folgende Lipide eingesetzt werden, seien sie Hydriert oder nicht, einzeln oder gemischt, bei jedem molaren Verhältnis: Distearoylphosphatidylcholin (DSPC), Phosphatidylcholin (PC), Cholesterol (Chol) oder Derivate, Sphingomielin (SM), Dioleoylphosphatidylcholin (DOPC), Dioleoylphosphatidylglycerol (DOPG), Phosphatidylglycerol (PG), Dimiristoylphosphatidylcholin (DMPC), Dipalmitoylphosphatidylcholin (DPPC), Gangliosiden, Ceramiden, Phosphatidylinositol (PI), Phosphatydinsäure, (PA), Dicetylphosphat (DcP), Dimiristoylphosphatidylglycerol (DMPG), Stearylamin (SA) Dipalmitoylphosphatidylglycerol (DPPG) und anderer synthetische Lipide.

15. Verfahren nach einem oder mehreren der Ansprüchen 3 bis 14, **dadurch gekennzeichnet, daß** das Dinitroanilin Trifluranin ist.

16. Liposomale Formulierungen nach einem oder mehreren der Ansprüchen 1 bis 2, wann nach einem Verfahren gemäß einem oder mehreren der Ansprüchen 3 bis 15 her gestellt wird.

17. Verwendung von Liposomale Formulierungen zur Herstellung von pharmazeutische Formulierung für Behandlung von Menschen und Tiere, Behandlung **dadurch gekennzeichnet, daß** eine therapeutisch wirksame Menge einer Liposomale Formulierung aus Dinitroanilin gemäß einem oder mehreren der Ansprüchen 1, 2 und 16 eigesetzt wird.

## Revendications

1. Formulation liposomiale qui contient une dinitroaniline incorporée ou encapsulée, par exemple trifluraline, pour utilisation dans la préparation d'une formulation pharmaceutique, **caractérisé en ce que** la dite formulation contient un mélange de deux populations distinctes de liposomes avec des particules aux diamètres moyens respectivement supérieur et inférieur à 100 nm.

2. Formulation liposomiale, selon la revendication 1, **caractérisé en ce qu'**on mélange des populations de particules, respectivement plus grandes que 400 nm et plus petites que 50 nm.

3. Procédé d'obtention d'une formulation liposomiale qui contient une dinitroaniline, composée de deux populations distinctes de liposomes de particules aux diamètres moyens respectivement supérieur et inférieur à 100 nm, ledit procédé comprenant les étapes suivantes:
• obtention des formulations liposomiales qui contiennent des vésicules de dinitroaniline par hydratation, avec une solution qui contient un agent antisublimation d'une pellicule lipidique qui contient la dinitroaniline
• obtention des différentes populations aux diamètres bien définis par une étape de dimensionnement
• mélange des populations distinctes obtenues;
• lyophilisation / déshydratation de la formulation liposomiale ainsi obtenue; et
• réhydratation de la formulation liposomiale déshydratée

4. Procédé selon la revendication 3, **caractérisé en ce qu'**on réalise l'étape de dimensionnement de la formulation liposomiale de dinitroaniline a fin de réduire le diamètre des vésicules, fait antérieurement à l'étape de déshydratation.

5. Procédé selon la revendication 4, **caractérisé en ce qu'**on réalise l'étape de dimensionnement par extrusion sous pression au travers des membranes poreuses.

6. Procédé selon l'une quelconque des revendications 3 à 5, **caractérisé en ce que** l'hydratation est effectuée par l'addition d'une petite quantité d'une solution aqueuse, suivie par l'addition du volume restant de la solution aqueuse, après une période de repos.

7. Procédé selon la revendication 6, **caractérisé en ce qu'**on réalise les étapes de réhydratation avec de la saccharose, tréhalose, glucose ou une autre solution de sucre quelconque.

8. Procédé selon l'une quelconque des revendications 3 à 7, **caractérisé en ce qu'**on mélange des populations de particules de diamètres différents.

9. Procédé selon la revendication 8, **caractérisé en ce qu'**on mélange des particules qui, après dimensionnement, présentent une population aux diamètres respectivement supérieurs et inférieurs à 100 nm.

10. Procédé selon la revendication 9, **caractérisé en ce qu'**on réalise l'étape de dimensionnement selon la revendication 5.

11. Procédé selon l'une quelconque des revendications 3 à 5 ou 9 à 10, **caractérisé en ce qu'**on réalise l'hydratation selon la revendication 6.

12. Procédé selon l'une quelconque des revendications 3 à 6 ou 9 à 11, **caractérisé en ce qu'**on utilise dans l'étape d'hydratation une solution selon la revendication 7.

13. Procédé selon l'une quelconque des revendications 3 à 7 ou 9 à 12, **caractérisé en ce qu'**on utilise des solutions selon la revendication 8.

14. Procédé selon l'une quelconque des revendications 3 à 13, **caractérisé en ce qu'**on utilise un quelconque des lipides suivants, hydrogenés ou pas, individuellement ou en mélange, dans un rapport molar quelconque: distéaroylphosphatidylcholine (DSPC), phosphatidylcholine (PC), cholestérol (Chol) ou ses dérivés, sphingomieline (SM), dioléoylphosphatidylcholine (DOPC), dioléoylphosphatidylglycérol (DOPG), phosphatidylglycérol (PG), dimyristoylphosphatidylcholine (DMPC), dipalmitoylphosphatidylcholine (DPPC), gangliosides, céramides, phosphatidylinositol (PI), acide phosphatydique, (PA), dicétylphosphate (DcP), dimyristoylphosphatidylglycérol (DMPG), stéarylamine (SA) dipalmitoylphosphatidylglycérol (DPPG) et d'autres lipides synthétiques.

15. Procédé selon l'une quelconque des revendications 3 à 14, **caractérisé en ce que** la dinitroaniline est la trifluraline.

16. Formulations liposomiales selon l'une quelconque des revendications 1 et 2, lorsqu'elles sont préparées par un procédé selon l'une quelconque des revendications 3 à 15.

17. Utilisation des formulations liposomiales pour la préparation d'une formulation pharmaceutique pour le traitement dans des,humains ou des animaux, **caractérisé par** l'utilisation d'une quantité terapeutique d'une formulation liposomiale de dinitroaniline selon l'une quelconque des revendications 1, 2 et 16.
